# FASCICULE DE BREVET EUROPEEN

(11) **EP 2 326 363 B1**
(45) Date de publication et mention de la délivrance du brevet: **14.10.2015**
(21) Numéro de dépôt: 09784198.5
(22) Date de dépôt: 08.06.2009
(51) Int. Cl.: B01D 61/24, B01D 63/08, A61M 1/02, A61M 1/36

(54) **UNITÉ DE FILTRATION D'UN FLUIDE MUNIE D'UNE INTERRUPTION DE JOINT**
FILTRATIONSGERÄT FÜR EINE FLÜSSIGKEIT MIT DICHTUNGSDISKONTINUITÄT
FILTRATION UNIT FOR A FLUID, HAVING A SEAL DISCONTINUITY

(30) Priorité: 27.06.2008 FR 0803651
(43) Date de publication de la demande: 01.06.2011
(73) Titulaire: MACO PHARMA, 59420 Mouvaux (FR)
(72) Inventeur: SUMIAN, Chryslain, 59130 Lambersart (FR); GODARD, David, 21000 Dijon (FR); CARLU, Matthieu, 59000 Lille (FR); VASSEUR, Ludovic, 59200 Tourcoing (FR); LENGLET, Mathieu, 62300 Lens (FR); CAPON, Nicolas, 59250 Halluin (FR)
(74) Mandataire: Sayettat, Julien Christian
(86) Numéro de dépôt international: PCT/FR2009/000673
(87) Numéro de publication internationale: WO 2010/004104

(56) Documents cités:
- EP-A- 0 953 361
- EP-A- 0 958 838
- WO-A-02/24256
- WO-A-90/15660
- FR-A- 2 774 293

## Description

L'invention concerne une unité de filtration d'un fluide biologique tel que le sang ou un composant sanguin, ainsi qu'un système à poches comprenant une telle unité de filtration.

L'invention s'applique à la filtration du sang ou d'un composant sanguin pour l'élimination de substances indésirables pour la transfusion, telles que les leucocytes, les pathogènes, les protéines telles que le prion, et/ou les substances utilisées dans des procédés d'inactivation et/ou d'élimination des pathogènes.

Le sang ou un composant sanguin, après son recueil et sa séparation dans le cas d'un composant, est notamment destiné à être transfusé à un patient qui en a besoin. Lors de cette transfusion, il est bien connu que les leucocytes sont indésirables en ce qu'ils sont susceptibles de provoquer chez le patient des réactions gênantes et/ou potentiellement dangereuses.

Il en est de même pour certaines substances tel que le prion, l'agent responsable des encéphalopathies spongiformes transmissibles, notamment de la variante de la maladie de Creutzfeldt-Jakob chez l'homme puisque des études ont montré qu'il existe un risque probable de transmission du prion lors de transfusions sanguines.

Pour éliminer ces substances indésirables, on connaît par exemple du document EP-A-526 678 des unités de filtration qui comprennent une enveloppe extérieure souple renfermant un milieu filtrant et munie d'au moins un port d'entrée et d'au moins un port de sortie entre lesquels le fluide à filtrer s'écoule suivant une direction. Le milieu filtrant délimite avec l'enveloppe extérieure un compartiment d'entrée pour le fluide à filtrer et un compartiment de sortie pour le filtrat. Ces unités comprennent un cadre souple permettant d'associer le média filtrant à l'enveloppe extérieure souple.

Ces unités de filtration souples sont fabriquées selon un procédé relativement complexe qui nécessite une intervention humaine. De plus, la présence d'un cadre souple augmente la quantité de matière première nécessaire et donc le prix de revient de telles unités.

Le document WO-A1-00/62891 propose une unité de filtration formée par assemblage de deux feuilles thermoplastiques souples sur lesquelles les ports d'entrée et sortie sont moulés par injection. Le média filtrant est directement soudé entre les deux feuilles, permettant une simplification du procédé de fabrication.

Une variante de cette unité de filtration est décrite dans le document WO-A1-01/91880. Dans ce document, les ports d'entrée et sortie sont moulés puis soudés sur la surface des feuilles de l'unité de filtration.

Cependant, dans ces deux derniers documents, les ports d'entrée et sortie disposés sur les faces de l'unité de filtration représentent un risque lors de la centrifugation, notamment celui d'endommager les poches en contact avec l'unité de filtration.

Dans le document WO-A1-01/56679, l'unité de filtration comprend deux feuilles souples entre lesquelles est disposé un milieu filtrant, un joint périphérique associant directement le milieu filtrant et les feuilles souples. Les ports d'entrée et sortie de cette unité sont disposés à la périphérie de l'unité de sorte qu'ils traversent le joint périphérique.

Le soudage simultané des feuilles, milieu filtrant et ports de cette unité de filtration représente une difficulté technique puisqu'il faut souder non seulement des matériaux de nature différente mais aussi de forme différente. Le risque de fuite au niveau des ports est donc important.

L'invention propose une unité de filtration dont la fabrication est automatisable. En outre, l'unité de filtration présente une performance d'écoulement améliorée, notamment au moment de l'amorçage de la filtration.

A cet effet et selon un premier aspect, l'invention propose une unité de filtration d'un fluide biologique tel que le sang ou un composant sanguin, comprenant une enveloppe extérieure formée de deux feuilles souples entre lesquelles le bord d'un milieu filtrant est associé le long d'un joint périphérique de sorte à former, de part et d'autre dudit milieu filtrant, un compartiment d'entrée pour le fluide à filtrer et un compartiment de sortie pour le filtrat, ladite unité de filtration comprenant en outre un port d'entrée en communication avec ledit compartiment d'entrée et un port de sortie en communication avec ledit compartiment de sortie, chacune des feuilles comprenant au moins une languette qui s'étend extérieurement depuis le joint périphérique, un port étant associé entre deux languettes de respectivement une feuille, et le joint périphérique présentant une interruption entre l'une desdites languettes et le milieu filtrant de sorte à permettre la communication entre le port et le compartiment.

Selon un deuxième aspect, l'invention propose un système à poches destiné à la filtration d'un fluide biologique comprenant :
- une unité de filtration selon le premier aspect,
- une première tubulure en communication avec d'une part l'orifice d'entrée de l'unité de filtration et d'autre part un moyen de mise en communication avec une source de fluide à filtrer,
- une poche de recueil de filtrat, et
- une deuxième tubulure en communication avec d'une part ladite poche de recueil du filtrat au niveau d'un orifice d'entrée et d'autre part l'orifice de sortie de l'unité de filtration.

D'autres objets et avantages apparaîtront au cours de la description qui suit.
Les figures 1 et 4 représentent de façon schématique la vue de face d'une unité de filtration selon deux variantes de l'invention dans lesquelles la tubulure d'entrée s'étend ou non jusqu'au voisinage du milieu filtrant.
La figure 2 représente de façon schématique la vue de dos de l'unité de filtration de la figure 1.
La figure 3 représente de façon schématique la vue en coupe de l'unité de filtration des figures 1 et 2.
Les figures 5 à 7 représentent de façon schématique les étapes d'un procédé pour décaler par rapport au plan de joint le port d'entrée ou de sortie de l'unité de filtration de la figure 1.
Les figures 8 et 9 représentent de façon schématique un système à poches en ligne et connectable respectivement, comprenant l'unité de filtration selon la figure 1.

Selon un premier aspect, l'invention propose une unité de filtration d'un fluide biologique tel que le sang ou un composant sanguin, tel qu'un concentré de globules rouges, un plasma ou un concentré plaquettaire.

Par exemple, l'unité de filtration est destinée à éliminer des substances cibles, notamment les leucocytes et/ou les prions, du sang total ou d'un composant sanguin.

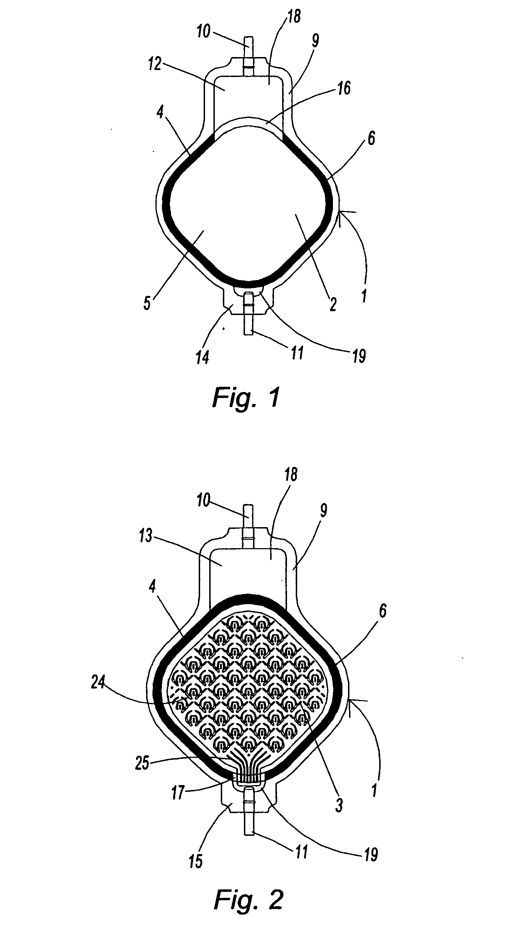

Selon les figures 1 à 4, l'unité de filtration 1 comprend une enveloppe extérieure formée de deux feuilles souples 2,3 entre lesquelles le bord 4 d'un milieu filtrant 5 est associé le long d'un joint périphérique 6 de sorte à former, de part et d'autre dudit milieu filtrant, un compartiment d'entrée 7 pour le fluide à filtrer et un compartiment de sortie 8 pour le filtrat.

Notamment, le compartiment d'entrée est formé entre l'une des feuilles 2 et l'amont du milieu filtrant 5, et le compartiment de sortie est formé entre l'autre feuille et l'aval du milieu filtrant 5.

Les termes amont et aval sont définis par rapport au sens d'écoulement du fluide lors de la filtration au travers l'unité de filtration.

Les feuilles souples 2,3 sont réalisées dans un matériau thermoplastique souple, tel que le polychlorure de vinyle. Le matériau thermoplastique est notamment stérilisable par chaleur ou vapeur.

Le milieu filtrant 5 comprend au moins une couche et avantageusement un assemblage de plusieurs couches filtrantes. Chacune des couches est sous forme d'une membrane poreuse, d'une mousse, d'un tissé ou d'un non-tissé. Notamment le milieu filtrant comprend plusieurs couches de non-tissé.

Le ou les matériau(x) formant la ou les couche(s) sont choisi(s) dans le groupe comprenant les polymères ou les copolymères à base de polypropylène, de polyester, de polyamide, de polyéthylène haute ou basse densité, de polyuréthanne, de fluorure de polyvinylidène, de polyvinylpyrrolidone et leurs dérivés. Avantageusement, le matériau est du polypropylène, du polyéthylène téréphtalate ou du polybutylène téréphtalate.

Selon une réalisation particulière, le milieu filtrant est apte à éliminer les leucocytes du sang. Un tel milieu filtrant correspond par exemple à l'élément poreux décrit dans le document EP-A1-1336417.

Le bord 4 du milieu filtrant 5 est associé entre les deux feuilles souples formant l'enveloppe de l'unité de filtration le long d'un joint périphérique 6.

Par exemple, l'association est réalisée par soudage, notamment soudage haute fréquence ou ultrasons.

Selon l'invention, chacune des feuilles 2,3 formant l'enveloppe comprend au moins une languette 12, 13, 14, 15 qui s'étend extérieurement depuis le joint périphérique 6, un port 10,11 étant associé entre deux languettes 12, 13, 14, 15 de respectivement une feuille 2,3.
Selon une première réalisation non représentée, le milieu filtrant et les feuilles sont associés le long du joint périphérique et les feuilles sont en outre associées entre elles sur leur périphérie le long d'un joint secondaire, distinct du joint périphérique, comme décrit dans le document WO-A1-01/91880.

Selon une réalisation plus avantageuse illustrée sur les figures 1 à 4, les languettes 12,13,14,15 sont associées entre elles sur leur périphérie le long d'un joint secondaire 9. En variante, le joint secondaire 9 s'étend en outre sur le joint périphérique 6 de sorte à associer les feuilles entre elles. Ainsi, le joint périphérique 6 et le joint secondaire 9 sont au moins en partie superposés. Ces joints périphérique 6 et secondaire 9 sont en particulier réalisés par soudage haute fréquence ou ultrasons. Le soudage des parties du joint périphérique 6 et du joint secondaire 9 qui sont superposées est avantageusement réalisé en une seule étape, c'est-à-dire que le joint périphérique 6 et le joint secondaire 9 sont confondus sur le bord du milieu filtrant.

Lorsque le milieu filtrant comprend plusieurs couches, celles-ci sont avantageusement d'abord soudées ensemble sur leur bord 4, et soudées ensuite aux feuilles souples 2,3 sur leur périphérie, les deux soudures étant au moins partiellement superposées et formant le joint périphérique 6.

Selon une variante, les feuilles souples 2,3 et le bord du milieu filtrant sont soudées en une seule fois de sorte à former le joint périphérique 6.

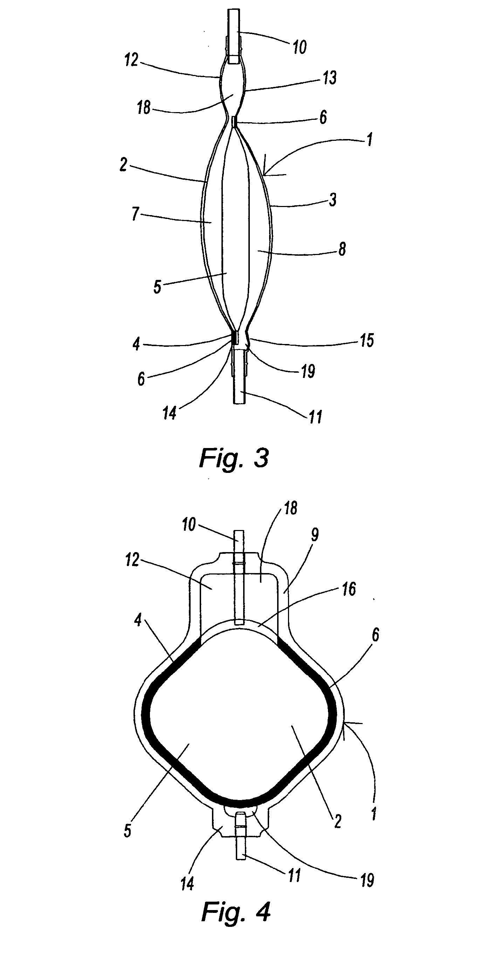

Selon la figure 3, l'unité de filtration comprend en outre un port d'entrée 10 en communication avec le compartiment d'entrée 7 et un port de sortie 11 en communication avec le compartiment de sortie 8.

Par exemple, le port d'entrée et/ou de sortie 10,11 est formé d'une portion de tubulure.

Selon l'invention, le port est associé entre deux languettes 12,13,14,15 de respectivement une feuille 2,3.

Ainsi, le port d'entrée 10 et/ou de sortie 11 ne traverse pas le joint périphérique 6. Autrement dit, la soudure au niveau de ce ou ces ports ne comprend pas le milieu filtrant 5. Par conséquent, l'assemblage des ports sur l'enveloppe est résistant puisque la soudure réalisée avec des matériaux de même nature, et le risque de fuite est quasi nul à ce niveau.

Pour permettre la communication entre le port d'entrée 10 ou de sortie 11 et le compartiment d'entrée 7 ou sortie 8, respectivement, le joint périphérique 6 présente une interruption 16,17 entre l'une desdites languettes 12,15 et le milieu filtrant. 5

Plus précisément, l'une desdites languettes 12,15 comprend une partie en regard du joint périphérique 6 qui est non associée au milieu filtrant 5. Les bords des languettes sont associés entre eux le long du joint secondaire 9.

L'interruption 16,17 du joint périphérique 6 est formée par exemple lors du soudage des bords 4 du milieu filtrant 5 avec les feuilles souples 2,3 en plaçant un doigt entre la partie de la languette 12,15 qui n'est pas soudée et le milieu filtrant.

Selon les figures 1 et 3 et de façon avantageuse, la feuille 2 formant le compartiment d'entrée 7, dite feuille d'entrée, comporte une languette supérieure 12 et une languette inférieure 14 opposée à la languette supérieure. La feuille 3 formant le compartiment de sortie 8, dite feuille de sortie, comporte une languette supérieure 13, et une languette inférieure 15 opposée à la languette supérieure 13. Les deux languettes supérieures 12,13 sont en regard l'une par rapport à l'autre de sorte à former une chambre d'entrée 18. De même, les deux languettes inférieures 14,15 sont en regard l'une par rapport à l'autre de sorte à former une chambre de sortie 19.

Les termes supérieur et inférieur sont définis en référence à l'unité de filtration dans sa position pendant la filtration par gravité d'un fluide.

L'unité 1 comprend une interruption 16 d'entrée pour permettre la communication entre le port d'entrée 10 et le compartiment d'entrée 7. De même, l'unité comprend une interruption 17 de sortie pour permettre la communication entre le port de sortie 10 et le compartiment de sortie 7.

Ainsi, l'unité de filtration peut facilement être fabriquée de façon automatisée en superposant simplement les différents composants, à savoir la feuille d'entrée 2, le port d'entrée 10, le milieu filtrant 5, le port de sortie 11 et la feuille de sortie 3, et en réalisant les joints périphérique 6 et secondaire 9.

Selon les figures 1 et 2, le port d'entrée 10 est associé entre les deux languettes supérieures 12,13 et le joint périphérique 6 présente une interruption 16 d'entrée entre la languette supérieure 12 de la feuille d'entrée 2 et le milieu filtrant 5. Par conséquent, l'interruption 16 d'entrée forme un passage entre le port d'entrée 10 et le compartiment d'entrée 7.

Ainsi, les deux ports 10,11 sont facilement soudables, par exemple par haute fréquence ou ultrasons aux feuilles formant l'enveloppe. En effet, la soudure ne comprend que des matériaux de même nature, notamment de même composition.

Au niveau de l'interruption 16 côté entrée, la partie de la languette supérieure 12 de la feuille d'entrée 2 en regard du joint périphérique 6 n'est pas associée au milieu filtrant 5. Par contre, la partie de la languette supérieure 13 de la feuille de sortie en regard du joint 6 est associé audit joint.

Le port de sortie 11 est associé entre les deux languettes inférieures 14,15 et le joint périphérique 6 présente une interruption 17 de sortie entre la languette inférieure 15 de la feuille de sortie 3 et le milieu filtrant 5. Par conséquent, l'interruption 17 de sortie forme un passage entre le port de sortie 11 et le compartiment de sortie 8.

Au niveau de l'interruption 17 côté sortie, la partie de la languette inférieure 15 de la feuille de sortie 3 en regard du joint périphérique 6 n'est pas associée au milieu filtrant 5. Par contre, la partie de la languette inférieure 14 de la feuille d'entrée en regard du joint 6 est associé audit joint.

Les languettes 12,13,14,15 entre lesquelles un port 10,11 est disposé, sont associées entre elles sur leur périphérie le long du joint secondaire 9, de sorte à former une chambre 18,19 en communication avec un compartiment 7,8. Ainsi, les deux languettes supérieures 12,13 entre lesquelles le port d'entrée 10 est associé forment la chambre d'entrée 18 en communication avec le compartiment d'entrée 7. Les deux languettes inférieures 14,15 entre lesquelles le port de sortie 11 est associé forment la chambre de sortie 19 en communication avec le compartiment de sortie 8.

Selon les figures 1 à 4, le port d'entrée et/ou de sortie 10,11 est associé entre les parties distales des languettes 12,13,14,15 par rapport à l'interruption 16,17 du joint périphérique 6.

Dans une première variante, la portion de tubulure d'entrée 10 et/ou de sortie 11 débouche au voisinage du milieu filtrant. Sur la figure 4, la portion de tubulure d'entrée 10 s'étend jusqu'au voisinage du milieu filtrant 5. Elle permet d'écarter la feuille d'entrée 2 du milieu filtrant 5 pour faciliter l'écoulement du fluide dans le compartiment d'entrée 7.

Avantageusement et comme représenté sur les figures 1 à 3, la portion de tubulure d'entrée 10 débouche au voisinage du joint secondaire 9, c'est-à-dire qu'elle ne s'étend pas substantiellement dans la chambre d'entrée 18. Durant la filtration, cette configuration évite de piéger du fluide à l'intérieur de la chambre 18. De plus, au début de la filtration, le fluide va s'accumuler dans cette chambre d'entrée 18. Ce réservoir de fluide va permettre d'homogénéiser le flux de fluide au travers du milieu filtrant.

De même, la portion de tubulure de sortie 11 débouche au voisinage du joint secondaire 9, c'est-à-dire qu'elle ne s'étend pas dans la chambre de sortie 19. Ainsi, l'unité ne comprend pas d'espace mort et les pertes sont réduites.

Le volume de la chambre d'entrée 18 est avantageusement supérieur à 5%, notamment supérieur à 10%, du volume du compartiment d'entrée. Ce volume est suffisant pour comprendre le volume d'air présent dans la tubulure connectée au port d'entrée 10 de l'unité de filtration 1. Ainsi, pendant la filtration, l'air est piégé dans la chambre d'entrée 18, ce qui permet d'utiliser la totalité de la surface du milieu filtrant 5.

Lors de la filtration, le fluide à filtrer s'écoule dans le compartiment d'entrée 7 de l'unité de filtration et remplit la chambre 18. La quantité de fluide en amont du milieu filtrant 5, dans le sens de l'écoulement des fluides, exerce une pression sur le milieu filtrant plus importante qu'en l'absence d'une telle chambre. L'amorçage est alors plus rapide.

De plus, l'air contenu dans la tubulure en amont de l'unité de filtration est poussé dans la chambre d'entrée par le fluide à filtrer. En fin de filtration, cet air va pousser le fluide vers la sortie de l'unité de filtration, améliorant la purge de l'unité de filtration et réduisant ainsi la perte de fluide.

Le volume de la chambre de sortie 19 est avantageusement inférieur à 50 %, notamment 10%, du volume du compartiment. En faisant varier la taille et la forme de la chambre de sortie, on peut créer un effet Venturi qui aide à la purge de l'unité de filtration. Cependant, cette chambre n'a pas un volume trop important pour ne pas augmenter le volume mort de l'unité de filtration.

Selon une réalisation particulière, les feuilles 2,3 sont associées entre elles suivant le plan du joint périphérique 6, et l'une des portions de tubulures 10,11 est décalée par rapport audit plan de joint vers le compartiment 7,8 avec lequel ledit port est en communication, comme décrit dans la demande de brevet FR 0703012.

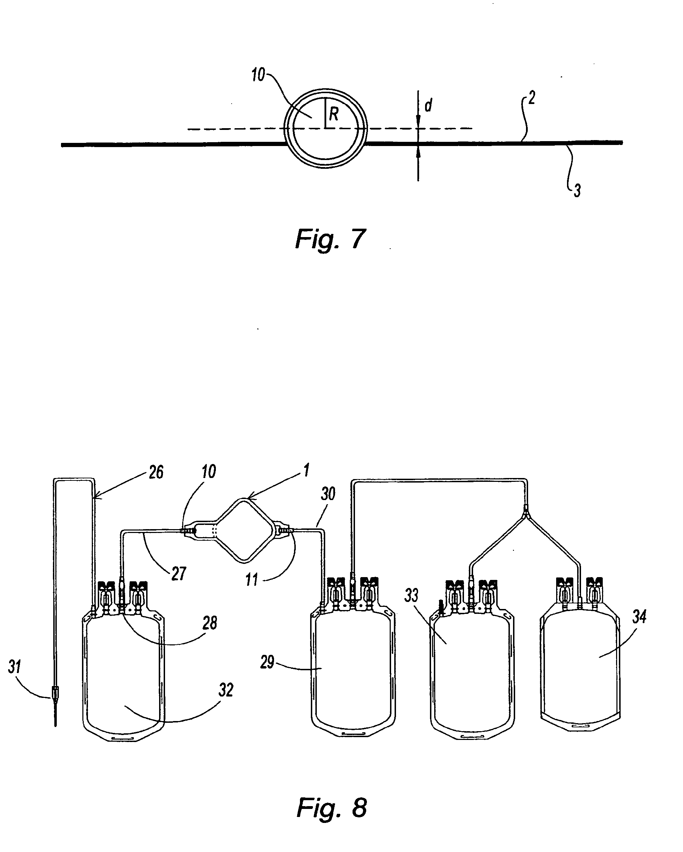

En particulier comme illustré sur la figure 7, la portion de tubulure 10,11 présente un rayon extérieur R et la distance de décalage d de ladite tubulure par rapport au plan de joint est strictement inférieure à R. Plus particulièrement la distance de décalage est comprise entre 20% et 80% du rayon R.

En particulier, les portions de tubulure d'entrée et sortie sont décalées par rapport au plan de joint.

Ainsi, et notamment dans le cas où la portion de tubulure s'étend jusqu'au voisinage du milieu filtrant, l'écoulement du fluide s'étend suivant une direction sensiblement parallèle au plan, permettant d'obtenir une meilleure distribution du flux sur le milieu filtrant.

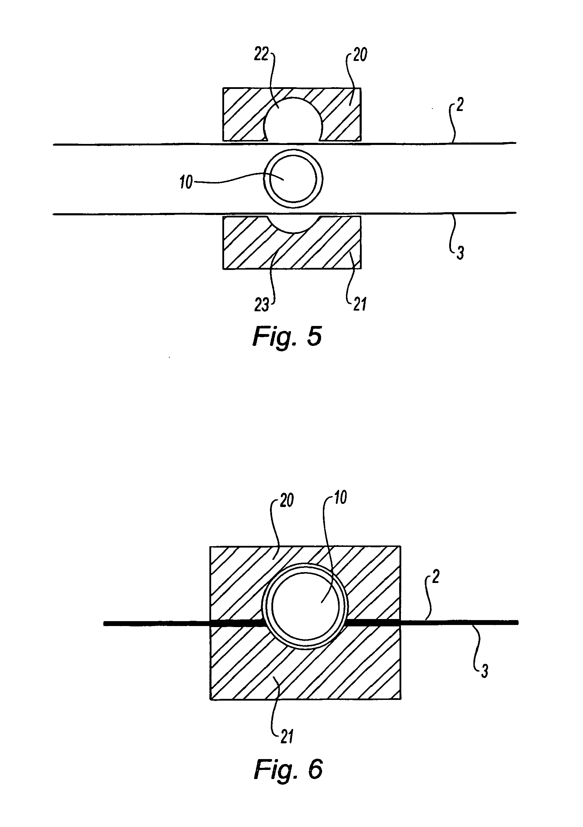

Comme représenté sur les figures 5 et 6, le décalage des portions de tubulures 10,11 est obtenu à l'aide de deux matrices 20,21 pourvues chacune d'au moins une empreinte 22,23, lesdites empreintes formant entre elles un logement de réception de la tubulure et étant dissymétriques.

Comme représenté sur la figure 2, au moins une partie de la surface interne de la feuille 3 délimitant le compartiment de sortie 8 est rugueuse. La rugosité empêche ladite feuille 3 et ledit milieu 5 de se collaber.

Avantageusement, seule la surface interne de la feuille de sortie 3 en regard du milieu filtrant 5 est rugueuse. Ainsi, la partie de la feuille associée au milieu filtrant le long du joint périphérique est lisse. La soudure étant réalisée sur une partie sans rugosité, elle est particulièrement solide.

En variante non représentée, au moins une partie de la surface interne de la feuille 2 délimitant le compartiment d'entrée 7 est rugueuse. Cette rugosité permet de canaliser et/ou de répartir le fluide à filtrer sur la totalité du milieu filtrant 5.

Plus particulièrement, la rugosité est formée par des aspérités 24 de profondeur comprise entre 0,1 et 3 mm.

Il est avantageux que la surface externe des feuilles d'entrée 2 et de sortie 3 présente une surface lisse. Cette surface lisse est nécessaire pour pouvoir imprimer du texte sur l'unité de filtration.

Comme représenté sur la figure 2, la rugosité au voisinage de la sortie de l'unité de filtration se présentent sous la forme d'une pluralité de canaux 25 convergeant vers le port de sortie. Ces canaux favorisent l'écoulement du fluide filtré vers la sortie de l'unité de filtration.

Selon un deuxième aspect et comme illustré sur les figues 8 et 9, l'invention concerne un système à poches 26 destiné à la filtration d'un fluide biologique comprenant
- une unité de filtration 1,
- une première tubulure 27 en communication avec d'une part l'orifice d'entrée 10 de l'unité de filtration 1 et d'autre part un moyen 28 de mise en communication avec une source de fluide à filtrer,
- une poche de recueil de filtrat 29, et
- une deuxième tubulure 30 en communication avec d'une part ladite poche de recueil du filtrat 29 au niveau d'un orifice d'entrée et d'autre part l'orifice de sortie 11 de l'unité de filtration.

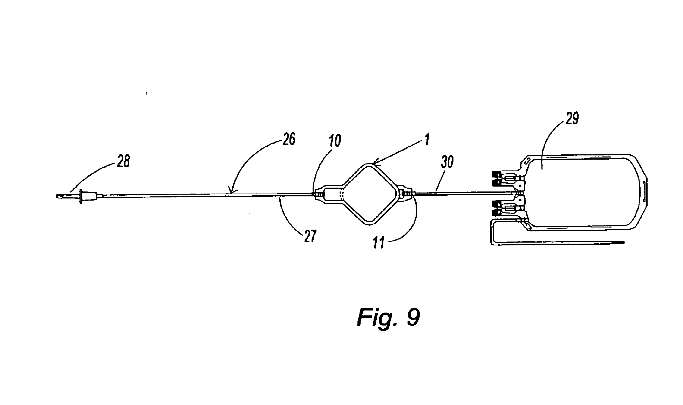

Le moyen de mise en communication du fluide à filtrer comprend une aiguille destinée à être insérée dans le bras d'un donneur. Selon une variante représentée sur la figure 9, le moyen de mise en communication est un perforateur destiné à être connecté à une poche contenant le fluide à filtrer.

Selon une autre variante représentée sur la figure 8, la poche 32 destinée à contenir le fluide à filtrer est connectée de fabrication à la première tubulure 27 et le moyen de communication comprend alors un orifice de sortie de ladite poche 32 destinée à contenir le fluide à filtrer. Le système à poches est dans ce cas un système clos.

La figure 8 représente plus particulièrement un exemple d'un système à poches utilisé pour filtrer le sang total issu d'un don et pour le séparer en plasma et concentré de globules rouges.

Le système comprend une aiguille 31 pour prélever le sang d'un donneur, une poche de recueil du sang total 32, une unité de filtration 1 telle que décrite précédemment destinée à filtrer les leucocytes du sang, une poche de recueil du filtrat 29, et deux poches satellites 33,34 destinées à recueillir le plasma et les globules rouges après centrifugation de la poche de recueil du filtrat. Les poches 29,31,33,34 et l'unité de filtration 1 sont reliées entre elles au moyen de tubulures souples et stérilisables.

## Revendications

1. Unité de filtration (1) d'un fluide biologique tel que le sang ou un composant sanguin, comprenant une enveloppe extérieure formée de deux feuilles souples (2,3) entre lesquelles le bord (4) d'un milieu filtrant (5) est associé le long d'un joint périphérique (6) de sorte à former, de part et d'autre dudit milieu filtrant, un compartiment d'entrée (7) pour le fluide à filtrer et un compartiment de sortie (8) pour le filtrat, ladite unité de filtration comprenant en outre un port d'entrée (10) en communication avec ledit compartiment d'entrée et un port de sortie (11) en communication avec ledit compartiment de sortie, ladite unité de filtration étant **caractérisée en ce que** chacune des feuilles (2,3) comprend au moins une languette (12,13,14,15) qui s'étend extérieurement depuis le joint périphérique (6), un port (10,11) étant associé entre deux languettes (12,13,1,4,15) de respectivement une feuille (2,3), et le joint périphérique (6) présentant une interruption (16,17) entre l'une desdites languettes (12,15) et le milieu filtrant (5) de sorte à permettre la communication entre le port (10,11) et le compartiment (7,8).

2. Unité de filtration selon la revendication 1, **caractérisée en ce que** l'une desdites languettes (12,15) comprend une partie en regard du joint périphérique (6) qui est non associée au milieu filtrant (5), de sorte à former l'interruption.

3. Unité de filtration selon la revendication 1 ou 2, **caractérisée en ce que** les languette (12,13,14,15) sont associées entre elles sur leur périphérie le long d'un joint secondaire (9).

4. Unité de filtration selon la revendication 3, **caractérisée en ce que** le joint secondaire (9) s'étend en outre sur le joint périphérique (6) de sorte à associer les feuilles entre elles.

5. Unité de filtration selon l'une quelconque des revendications 1 à 4, **caractérisée en ce qu'**elle comprend une interruption d'entrée (16) pour permettre la communication entre le port d'entrée (10) et le compartiment d'entrée (7).

6. Unité de filtration selon la revendication 5, **caractérisée en ce qu'**elle comprend deux languettes supérieures (12,13) entre lesquelles le port d'entrée (10) est associé, lesdites languettes formant une chambre d'entrée (18) dont le volume est supérieur à 5%.

7. Unité de filtration selon l'une quelconque des revendications 1 à 6, **caractérisée en ce qu'**elle comprend une interruption de sortie (17) du joint périphérique pour permettre la communication entre le port de sortie (11) et le compartiment de sortie (8).

8. Unité de filtration selon la revendication 7, **caractérisée en ce qu'**elle comprend deux languettes inférieures (14,15) entre lesquelles le port de sortie (11) est associé, lesdites languettes formant une chambre de sortie (19) dont le volume est inférieur à 50% du volume du compartiment de sortie (8).

9. Unité de filtration selon l'une quelconque des revendications 1 à 8, **caractérisée en ce que** le port d'entrée et/ou de sortie (10,11) est formé d'une portion de tubulure.

10. Unité de filtration selon la revendication 9, **caractérisée en ce que** la portion de tubulure (10,11) débouche au voisinage du milieu filtrant (5).

11. Unité de filtration selon l'une quelconque des revendications 1 à 10, **caractérisée en ce qu'**au moins une partie de la surface interne d'au moins une des feuilles (2,3) est rugueuse.

12. Unité de filtration selon la revendication 11, **caractérisée en ce que** la rugosité est formée par des aspérités (24) de profondeur comprise entre 0,1 et 3 mm.

13. Unité de filtration selon l'une quelconque des revendications 1 à 12, **caractérisée en ce que** le milieu filtrant (5) comprend un assemblage de plusieurs couches filtrantes.

14. Système à poches (26) destiné à la filtration d'un fluide biologique **caractérisé en ce qu'**il comprend
- une unité de filtration (1) selon l'une quelconque des revendications 1 à 13,
- une première tubulure (27) en communication avec d'une part l'orifice d'entrée (10) de l'unité de filtration et d'autre part un moyen de mise en communication (28) avec une source de fluide à filtrer,
- une poche de recueil de filtrat (29),
- une deuxième tubulure (30) en communication avec d'une part ladite poche de recueil du filtrat (29) au niveau d'un orifice d'entrée et d'autre part l'orifice de sortie de l'unité (11) de filtration.

## Patentansprüche

1. Filtrationseinheit (1) für eine biologische Flüssigkeit, wie Blut oder ein Blutbestandteil, wobei die Filtrationseinheit eine äußere Hülle umfasst, die von zwei flexiblen Folien (2, 3) gebildet wird, zwischen denen der Rand (4) eines Filtermediums (5) entlang einer Umfangsdichtung (6) derart verbunden ist, dass auf beiden Seiten des besagten Filtermediums einen Einlassraum (7) für die zu filtrierende Flüssigkeit und einen Auslassraum (8) für das Filtrat gebildet werden, wobei die besagte Filtrationseinheit außerdem einen Einlassanschluss (10) in Kommunikation mit dem besagten Einlassraum und einen Auslassanschluss (11) in Kommunikation mit dem besagten Auslassraum umfasst, wobei die besagte Filtrationseinheit **dadurch gekennzeichnet ist, dass** jede der Folien (2, 3) mindestens eine Lasche (12, 13, 14, 15) umfasst, die sich außerhalb von der Umfangsdichtung (6) erstreckt, ein Anschluss (10, 11) zwischen zwei Laschen (12, 13, 1,4, 15) von jeweils einer Folie (2, 3) verbunden ist und die Umfangsdichtung (6) eine Unterbrechung (16, 17) zwischen einer der besagten Laschen (12, 15) und dem Filtermedium (5) aufweist, um die Kommunikation zwischen dem Anschluss (10, 11) und dem Raum (7, 8) zu ermöglichen.

2. Filtrationseinheit nach Anspruch 1, **dadurch gekennzeichnet, dass** eine der besagten Laschen (12, 15) einen Teil in Bezug auf die Umfangsdichtung (6) umfasst, der nicht mit dem Filtermedium (5) verbunden ist, um die Unterbrechung zu bilden.

3. Filtrationseinheit nach Anspruch 1 oder 2, **dadurch gekennzeichnet, dass** die Laschen (12, 13, 14, 15) auf ihrem Umfang entlang einer sekundären Dichtung (9) miteinander verbunden sind.

4. Filtrationseinheit nach Anspruch 3, **dadurch gekennzeichnet, dass** die sekundäre Dichtung (9) sich auch auf der Umfangsdichtung (6) erstreckt, um die Folien miteinander zu verbinden.

5. Filtrationseinheit nach einem der Ansprüche 1 bis 4, **dadurch gekennzeichnet, dass** sie eine Einlassunterbrechung (16) umfasst, um die Kommunikation zwischen dem Einlassanschluss (10) und dem Einlassraum (7) zu ermöglichen.

6. Filtrationseinheit nach Anspruch 5, **dadurch gekennzeichnet, dass** sie zwei obere Laschen (12, 13) umfasst, zwischen denen der Einlassanschluss (10) verbunden ist, wobei die besagten Laschen eine Einlasskammer (18) bilden, deren Volumen mehr als 5 % beträgt.

7. Filtrationseinheit nach einem der Ansprüche 1 bis 6, **dadurch gekennzeichnet, dass** sie eine Auslassunterbrechung (17) der Umfangsdichtung umfasst, um die Kommunikation zwischen dem Auslassanschluss (11) und dem Auslassraum (8) zu ermöglichen.

8. Filtrationseinheit nach Anspruch 7, **dadurch gekennzeichnet, dass** sie zwei untere Laschen (14, 15) umfasst, zwischen denen der Auslassanschluss (11) verbunden ist, wobei die besagten Laschen eine Auslasskammer (19) bilden, deren Volumen weniger als 50 % des Volumens des Auslassraums (8) beträgt.

9. Filtrationseinheit nach einem der Ansprüche 1 bis 8, **dadurch gekennzeichnet, dass** der Einlassanschluss und/oder der Auslassanschluss (10, 11) von einem Leitungsabschnitt gebildet werden.

10. Filtrationseinheit nach Anspruch 9, **dadurch gekennzeichnet, dass** der Leitungsabschnitt (10, 11) in der Nähe des Filtermediums (5) mündet.

11. Filtrationseinheit nach einem der Ansprüche 1 bis 10, **dadurch gekennzeichnet, dass** mindestens ein Teil der Innenfläche mindestens einer der Folien (2, 3) rau ist.

12. Filtrationseinheit nach Anspruch 11, **dadurch gekennzeichnet, dass** die Rauheit von Unebenheiten (24) mit einer Tiefe, die zwischen 0,1 und 3 mm liegt, gebildet wird.

13. Filtrationseinheit nach einem der Ansprüche 1 bis 12, **dadurch gekennzeichnet, dass** das Filtermedium (5) einen Verbund von mehreren Filterlagen umfasst.

14. Beutelsystem (26), das zur Filtration einer biologischen Flüssigkeit vorgesehen ist, **dadurch gekennzeichnet, dass** das Beutelsystem Folgendes umfasst:
- eine Filtrationseinheit (1) nach einem der Ansprüche 1 bis 13,
- eine erste Leitung (27) in Kommunikation mit einerseits der Einlassöffnung (10) der Filtrationseinheit und andererseits einem Mittel zur Herstellung einer Kommunikation (28) mit einer Quelle einer zu filtrierenden Flüssigkeit,
- einen Filtratsammelbeutel (29),
- eine zweite Leitung (30) in Kommunikation mit einerseits dem besagten Filtratsammelbeutel (29) auf Höhe einer Einlassöffnung und andererseits der Auslassöffnung (11) der Filtrationseinheit.

## Claims

1. Filtration unit (1) for a biological fluid such as blood or a blood component, comprising an outer casing formed by two flexible sheets (2, 3), between which the edge (4) of a filtering medium (5) is connected along a peripheral seal (6) so as to form, on either side of said filtering medium, an inlet compartment (7) for the fluid to be filtered and an outlet compartment (8) for the filtrate, said filtration unit also comprising an inlet port (10) that communicates with said inlet compartment and an outlet port (11) that communicates with said outlet compartment, said filtration unit being **characterised in that** each of the sheets (2, 3) comprises at least one flap (12, 13, 14, 15) which extends externally from the peripheral seal (6), a port (10, 11) being connected between two flaps (12, 13, 1,4, 15) of a sheet (2, 3) respectively, and the peripheral seal (6) having a break (16, 17) between one of said flaps (12, 15) and the filtering medium (5) so as to enable communication between the port (10, 11) and the compartment (7, 8).

2. Filtration unit according to claim 1, **characterised in that** one of said flaps (12, 15) has a section opposite the peripheral seal (6) which is not connected to the filtering medium (5), so as to form the break.

3. Filtration unit according to either claim 1 or claim 2, **characterised in that** the flaps (12, 13, 14, 15) are connected to one another on their periphery along a secondary seal (9).

4. Filtration unit according to claim 3, **characterised in that** the secondary seal (9) also extends over the peripheral seal (6) so as to join the sheets together.

5. Filtration unit according to any one of claims 1 to 4, **characterised in that** it comprises an inlet break (16) to allow communication between the inlet port (10) and the inlet compartment (7).

6. Filtration unit according to claim 5, **characterised in that** it comprises two upper flaps (12,13) between which the inlet port (10) is connected, said flaps forming an inlet chamber (18) the volume of which is greater than 5%.

7. Filtration unit according to any one of claims 1 to 6, **characterised in that** it comprises an outlet break (17) of the peripheral seal to allow communication between the outlet port (11) and the outlet compartment (8).

8. Filtration unit according to claim 7, **characterised in that** it comprises two lower flaps (14, 15) between which the outlet port (11) is connected, said flaps forming an outlet chamber (19) the volume of which is less than 50% of the volume of the outlet compartment (8).

9. Filtration unit according to any one of claims 1 to 8, **characterised in that** the inlet port and/or the outlet port (10, 11) is formed by a portion of pipe.

10. Filtration unit according to claim 9, **characterised in that** the portion of pipe (10, 11) opens in the vicinity of the filtering medium (5).

11. Filtration unit according to any one of claims 1 to 10, **characterised in that** at least a part of the internal surface of at least one of the sheets (2, 3) is roughened.

12. Filtration unit according to claim 11, **characterised in that** the roughness is formed by roughened areas (24) with a depth of between 0.1 and 3 mm.

13. Filtration unit according to any one of claims 1 to 12, **characterised in that** the filtering medium (5) comprises an assembly of several filtering layers.

14. System of pockets (26) designed for filtering a biological fluid **characterised in that** it comprises
- a filtration unit (1) according to any one of claims 1 to 13,
- a first pipe (27) which communicates on the one hand with the inlet opening (10) of the filtration unit and on the other hand with a means of communication (28) with a source of fluid to be filtered,
- a pocket for collecting filtrate (29),
- a second pipe (30) that communicates on the one hand with said pocket for collecting filtrate (29) at the level of an inlet opening and on the other hand with the outlet opening (11) of the filtration unit.
